# EUROPÄISCHE PATENTSCHRIFT

(11) **EP 1 594 885 B1**
(45) Veröffentlichungstag und Bekanntmachung des Hinweises auf die Patenterteilung: **07.01.2009**
(21) Anmeldenummer: 04709952.8
(22) Anmeldetag: 11.02.2004
(51) Int. Cl.: C07J 63/00, A61K 31/56, A61P 35/00

(54) **MEDIKAMENT ZUR WACHSTUMSINHIBIERUNG VON TUMOREN**
MEDICAMENT FOR INHIBITING TUMOUR GROWTH
MEDICAMENT POUR INHIBER LA CROISSANCE DE TUMEURS

(30) Priorität: 11.02.2003 AT 2002003
(43) Veröffentlichungstag der Anmeldung: 16.11.2005
(73) Patentinhaber: Novelix Pharmaceuticals, Inc., Pasadena, CA 91101 (US)
(72) Erfinder: SELZER, Edgar, A-3423 ST. Andrä-Wördern (AT); JANSEN, Burkhard, Pasadena, CA 91107 (US); PASCHKE, Reinhard, BioService Halle GmbH, 06120 Halle (DE)
(74) Vertreter: Sonn & Partner Patentanwälte
(86) Internationale Anmeldenummer: PCT/AT2004/000045
(87) Internationale Veröffentlichungsnummer: WO 2004/072092

(56) Entgegenhaltungen:
- WO-A-96/29068
- US-A- 5 962 527
- US-A1- 2002 052 352
- C. WERMUTH: "The Practice of Medicinal Chemistry" 1996 , ACADEMIC PRESS , SAN DIEGO, CA 92101 XP002277818 Seite 756 -Seite 776

## Beschreibung

Die vorliegende Erfindung betrifft neue Derivate der Betulinsäure mit verstärkter Wirksamkeit zur Behandlung von Karzinomen und HIV-Erkrankung, ein Verfahren zur Herstellung derartiger neuer Derivate der Betulinsäure sowie ihre Verwendung als Pharmazeutika. Die erfindungsgemäßen Verbindungen können in Analogie zur Betulinsäure beim Menschen wie auch in Tieren zur Inhibierung des Wachstums verschiedener Tumore (Melanome, Sarkome, Lymphome, Plattenepithelkarzinomen sowie weitere nachstehend angeführte Tumore) zum klinischen Einsatz kommen sowie für die Behandlung von HIV-Erkrankungen und - wegen ihrer antiphlogistischen Wirkungen - bei unspezifischen entzündlichen Erkrankungen verwendet werden.

Die Erfindung betrifft neue Derivate der Betulinsäure mit der allgemeinen Formel (I) 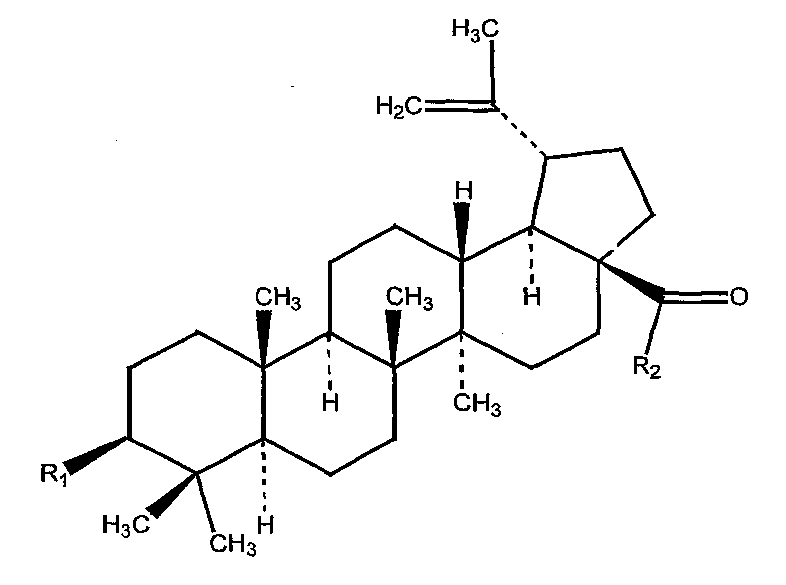 worin R₁ eine Hydroxygruppe, eine Aminogruppe, eine geschützte Hydroxygruppe oder eine geschützte Aminogruppe darstellt. Geeignete Schutzgruppen sind im Stand der Technik bekannt, z.B. in Kapitel 2 und 7 von "Protective Groups in Organic Synthesis", T.W. Greene und P.G.M. Wuts, 3. Auflage, John Wiley & Sons, Inc. (1999), diese Offenbarung wird hiermit durch ausdrückliche Bezugnahme in die vorliegende Beschreibung mit aufgenommen, und R₂ =

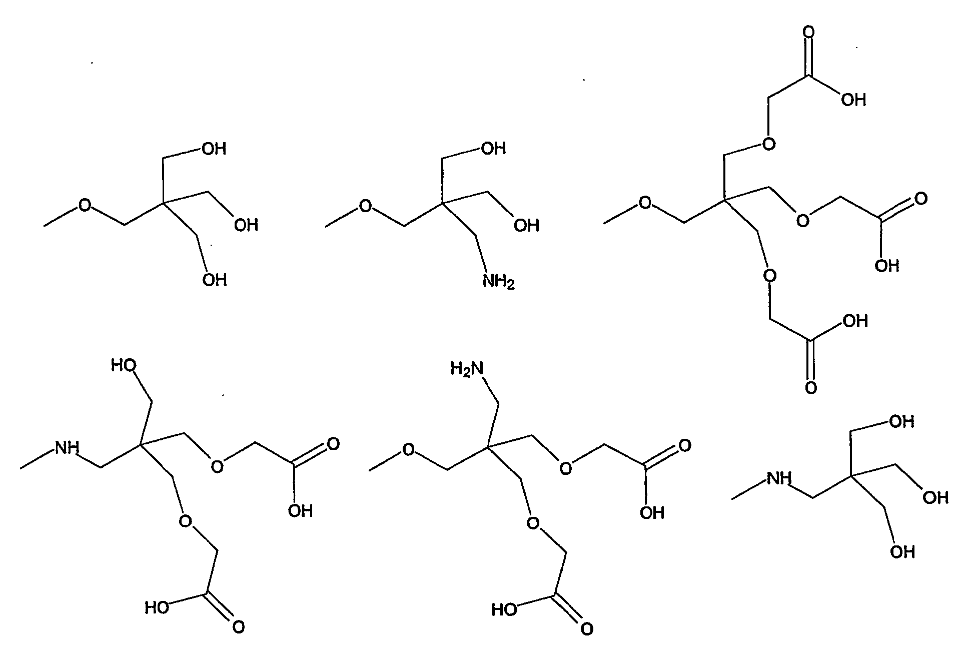

Insbesondere betrifft die vorliegende Erfindung neue Derivate der Betulinsäure mit der allgemeinen Formel (I) wie oben angegeben, worin R₁ eine Hydroxygruppe, eine Aminogruppe oder eine der folgenden geschützten Hydroxy- oder Aminogruppen ist: 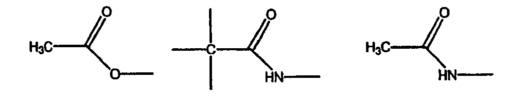 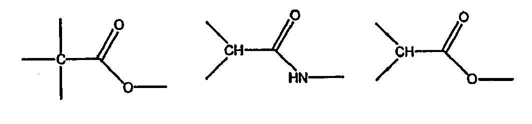 und R₂ die oben angegebene Bedeutung hat.

Bei den erfindungsgemäßen Verbindungen handelt es sich um neue Derivate der Betulinsäure mit höherer Wirksamkeit sowie besserer Löslichkeit in polaren Lösungsmitteln und damit bedeutend besseren Einsatzmöglichkeiten. Selbstverständlich fallen auch etwaige Salze und Einschlussverbindungen der erfindungsgemäßen Verbindung unter die oben angegebene Definition der allgemeinen Formel (I).

Weiters betrifft die vorliegende Erfindung ein Verfahren zur Herstellung von Verbindungen der allgemeinen Formel (I), wobei ein durch den Substituenten R₁ entsprechend geschütztes Betulinsäurehalogenid, insbesondere ein Betulinsäurechlorid, mit einem zur Vorsehung des Substituenten R₂ entsprechend substituierten Alkohol oder Amin umgesetzt wird. Die derart erhaltene Verbindung der allgemeinen Formel (I), worin R₁ eine geschützte Hydroxy- oder Aminogruppe darstellt, kann dann gewünschtenfalls in Abhängigkeit von der gewählten Schutzgruppe durch im Stand der Technik bekannte Massnahmen entschützt werden, um eine Verbindung der allgemeinen Formel (I) vorzusehen, worin R₁ Hydroxy oder Amino bedeutet.

Der Naturstoff Betulinsäure, welcher als Ausgangs- und Vergleichsverbindung für die erfindungsgemäßen Verbindungen dient, ist ein Triterpen, welches bereits Anfang des letzten Jahrhunderts isoliert wurde. Der Name leitet sich vom entsprechenden Alkohol Betulin, einem Inhaltsstoff der Birkenrinde, ab, wo er in großen Mengen vorkommt. Betulinsäure besitzt anti-Malaria, anti-entzündliche, anti-HIV und anti-Tumor Effekte, die in einer Vielzahl verschiedener Publikationen beschrieben sind. Als Chemotherapeutikum verwendet induziert Betulinsäure in Tumorzellen verschiedenen Ursprungs (z.B. in Melanomzellen) den sogenannten progammierten Zelltod, auch Apoptose genannt. In Melanomen wurden in humanen Mausmodellen sowohl in vitro als auch in vivo antiproliferative Effekte nachgewiesen. Insbesondere in Melanomzellen scheint die Betulinsäure spezifisch in Tumorzellen einen apoptotischen Zelluntergang herbeizuführen, während Melanozyten dieser Substanz gegenüber weitgehend resistent sind. Bisher sind kaum Daten über mögliche synergistische Effekte zwischen der Betulinsäure und anderen Zytostatika bekannt. Besonders gut wurden die molekularen Wirkungsmechanismen bei kindlichen Tumoren (Ewing Sarkom, Medulloblastom) sowie beim Glioblastom durch die Forschungsgruppe um S. Fulda (Kinderklinik - Ulm) untersucht.

Weitere bekannte und in der wissenschaftlichen Literatur gut dokumentierte Effekte der Betulinsäure und ihrer bekannten Derivate sind ihre Wirksamkeit gegen HIV-Viren, deren Replikation wie auch deren Bindung am Rezeptor sie unterdrücken können, wie auch ihre anti-entzündliche Wirksamkeit, die z.B. in einem Maus-Ohr Entzündungsmodell beschrieben ist. Betulinsäure ist somit aufgrund ihrer - im Tierversuch und in der Zellkulturen beschriebenen - antiproliferativen Wirkung gegen verschiedene Tumore (Melanome, neuroektodermale Tumore, Sarkome) eine ausgesprochen interessante Substanz für eine alleinige wie auch für mögliche Kombinationstherapien mit anderen zytostatisch wirksamen Substanzen und Substanzen die den Zelltod modulieren können, z.B. Antisensoligonukleotide gegen verschiedene anti-apoptotische Bcl-2 Familienmitglieder, insbesondere Bcl-2, Bcl-xL sowie Mcl-1.

Publikationen über den Wirkungsmechanismus von Betulinsäure hauptsächlich in Melanomen sowie beim Ewing-Sarkom, Glioblastom und Medulloblastom haben gezeigt, dass ihre Effekte zu einem wesentlichen Teil durch Induktion von Apoptose auf mitochondrialer Ebene verursacht ist. Unklar zum jetzigen Zeitpunkt ist, welche primären Angriffspunkte sie in der Zelle hat, unter anderem sind weder die Bindungsstellen (Rezeptoren), falls vorhanden, noch die initialen Signalwege ausreichend erforscht. Die Erfinder und andere Autoren konnten jedoch zeigen, dass Betulinsäure Apoptose in malignen Zellen induziert, jedoch humane Melanozyten und auch normale Zellen weniger sensibel als die malignen Zellen zu sein scheinen. Diese Beobachtung ist vor allem auch deswegen von Interesse, da tierexperimentelle Daten in der Maus keine nennenswerte Toxizität gezeigt haben. Abgesehen von diesen, in der Zellkultur erhobenen Daten, gibt es Hinweise darauf, dass sich Betulinsäure stärker in Tumorgeweben als in Normalgeweben anreichert. Es wurde auch die Induktion verschiedener Bcl-2 Familienmitglieder in Melanomzellen wie auch in normalen Melanozyten und in Sarkomlinien untersucht. Dabei stellte sich heraus dass die Expression des antiapoptotischen Proteins Mcl-1 durch Betulinsäure innerhalb weniger Stunden induziert werden kann. Die anderen untersuchten Proteine dieser Genfamilie, vor allem Bcl-2 & Bcl-x, wie auch die Expression des p53 Proteins blieben unter dieser Behandlung unverändert. Die Daten aus der wissenschaftlichen Literatur weisen darauf hin, dass die Effekte der Betulinsäure unabhängig vom p53-Protein sind. Da kürzlich gezeigt wurde, dass Bcl-2 wie auch Bcl-xL die Betulinsäure-induzierte Apoptose hemmen können, legen diese Beobachtungen eine Kombination von Betulinsäure mit einer Bcl-2 und/oder Bcl-xL Antagonisierung, z.B. durch Antisensoligonukleotide (ASO), nahe. Die selben Überlegungen gelten für mögliche Kombinationen mit z.B. Mcl-1 ASO's. Eine Eigenschaft von Betulinsäure von potentieller klinischer Relevanz ist die rezente Beobachtung, dass ihre Zytotoxizität durch niedrige pH-Werte im Kulturmedium verstärkt wird. Der pH-Wert ist in vielen Tumoren niedriger als in Normalgeweben (Noda Y, Kaiya T, Kohda K, Kawazoe Y., "Enhanced cytotoxicity of some triterpenes toward leukemia L1210 cells cultured in low pH media: possibility of a new mode of cell killing": Chem Pharm Bull (Tokyo). 1997 Oct;45(10):1665-70.). Dieselben Autoren fanden, dass Betulinsäure aktiver gegen ruhende Zellen als gegen Zellen in der Wachstumsphase ist. Diese Eigenschaft könnte von zusätzlicher Bedeutung beim klinischen Einsatz sein, da viele Chemotherapeutika wie Radiotherapie weniger effektiv gegenüber ruhende und/oder acidotische Zellpopulationen sind.

Zum jetzigen Zeitpunkt sind vor allem Melanome, neuroektodermale Tumore wie auch Sarkome und HIV am besten untersucht. Diese sind besonders schwierig zu behandelnde Tumore, bei denen vor allem die generalisierte Form der Erkrankung wenig erfolgversprechende Behandlungsoptionen aufweist. Bei Patienten mit metastasiertem Melanom sind die Therapiemöglichkeiten weitgehend auf einige wenige Substanzen limitiert. Dazu zählt 5-(3,3-Dimethyl-1-triazenyl)-1-H-imidaz-ole-4-carboxamid (Dacarbazin, DTIC). Dacarbazin ist nach wie vor die effektivste Monotherapie für das Melanom mit Ansprechraten in der Größenordnung von ca. 30 %. Kombinationstherapien mit weiteren synthetischen oder rekombinanten Substanzen wie z.B. BCNU, Cisplatin, Tamoxifen, Interferon-Alpha und Interleukin-2 zeigen höhere Ansprechraten in einigen klinischen Studien. Diese sind jedoch zeitlich limitiert und gehen mit einer erhöhten Toxizität einher. Einige Substanzen die von natürlichen Produkten abstammen, wie z.B. Adriamycin, Bleomycin, Etoposid, und andere, sind bezüglich ihrer Wirksamkeit gegen Melanome und ihrer Toxizität untersucht worden. Keine dieser Substanzen konnte jedoch letztendlich im klinischen Alltag überzeugen.

Das erfindungsgemäße Verfahren wird im folgenden anhand von einigen Beispielen näher erläutert, wobei sich die Offenbarung der Erfindung nicht auf diese Beispiele beschränkt.

### 1) Acetylbetulinsäure - 2-amino-3-hydroxy-2-hydroxymethyl-propylester IV (Verbindung B)

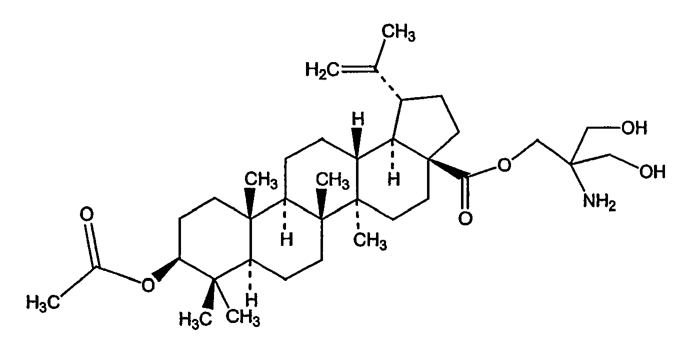

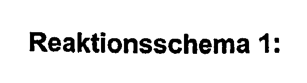

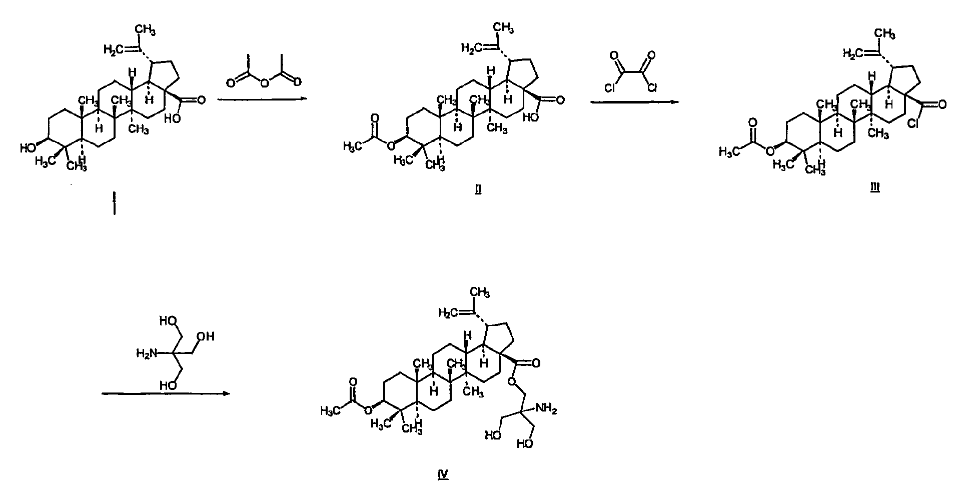

Die Synthese des Acetylbetulinsäure-2-amino-3-hydroxy-2-hydroxymethyl propylesters IV erfolgt ausgehend von der Betulinsäure I über die Zwischenstufen der Acetylbetulinsäure II und des entsprechenden Säurechlorids III, durch Umsetzung des Säurechlorids III mit Trishydroxymethylaminomethan.

### a) Acetylbetulinsäure (Mw 498,74) II

2 g Betulinsäure I (Mw 456,70) werden 2 Stunden in 50 ml Acetanhydrid am Rückfluss erhitzt. Nach dem Abkühlen gibt man den Reaktionsansatz unter starkem Rühren in Eiswasser, filtriert ab und wäscht den erhaltenen Feststoff mit Wasser bis der Essigsäuregeruch verschwunden ist.

Der Feststoff wird sodann in 70%igem Ethanol 4 Stunden unter Rühren am Rückfluss erhitzt.

Nach dem Abkühlen der Reaktionslösung wird filtriert, die Mutterlauge etwas eingeengt, im Eisbad abgekühlt und nochmals filtriert. Ausbeute 86%, Fp: 290°C.

### b) Acetylbetulinsäurechlorid (Mw 517,18) III

2 g Acetylbetulinsäure II werden in trockenem Benzen (35 ml) vorgelegt und ein 10facher Überschuss an Oxalylchlorid (3,4 ml) wird zugegeben. Das Reaktionsgemisch wird 8 Stunden unter Kühlung gerührt und anschließend das Lösungsmittel sowie das überschüssige Oxalylchlorid am Rotationsverdampfer abgezogen. Um Reste des Oxalylchlorids zu entfernen, werden nochmals 20 ml Benzen zugegeben und wiederum im Vakuum abgezogen.

### c) Acetylbetulinsäure - 2-amino-3-hydroxy-2-hydroxymethyl-propylester (Mw 601, 86) IV, (Verbindung B)

Das aus 1 g Acetylbetulinsäure ( ca. 0,002 mol ) nach dem Verfahren gemäß b) erhaltene Säurechlorid wird ohne weitere Reinigung umgesetzt. Dazu wird es in 35 ml Dioxan (trocken) gelöst und Tris-(hydroxymethyl)-aminomethan wird im doppelten Überschuss (0,004 mol, 0,5 g) zugegeben. Nach Zugabe von einer Spatelspitze DMAP und 3 Tropfen Pyridin wird das Reaktionsgemisch für 2 Tage bei Raumtemperatur gerührt.

Anschließend wird vom Feststoff abfiltriert, die Lösung am Rotationsverdampfer eingeengt und in Chloroform aufgenommen. Diese Chloroformlösung wird mehrmals mit 1%iger Salzsäure, Wasser und gesättigter Kochsalzlösung pyridinfrei gewaschen. Nach Trocknung über Na₂SO₄ wird das Lösungsmittel abgezogen und das Produkt über eine Kieselgelsäule oder (und) Chromatotron gereinigt. Als Laufmittel wurde ein Chloroform-Methanol-Gemisch im Verhältnis 10:1 eingesetzt. Ausbeute 20%, Fp.: 156°C.

### 2) Acetylbetulinsäure- N-(1,1-bis(hydroxymethyl)-2-hydroxyethyl)formamid (Mw 601,86), (Verbindung C)

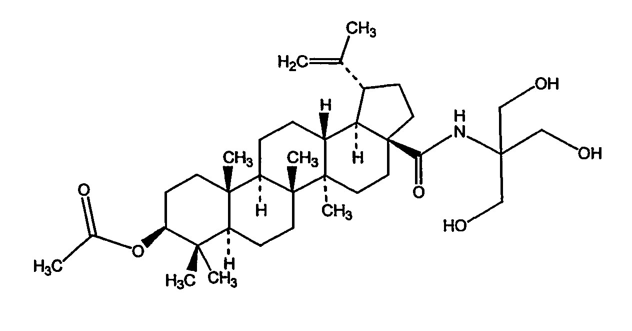

Die Synthese des Acetylbetulinsäure-N-(1,1-bis(hydroxymethyl)-2-hydroxyethyl)-formamids (Verbindung C) erfolgt in Analogie zum Reaktionsschema 1 ausgehend von der Betulinsäure I über die Zwischenstufen der Acetylbetulinsäure II und des entsprechenden Säurechlorids III, durch Umsetzung des Säurechlorids III mit Tris-(hydroxymethyl)-aminomethan.

Das aus 1g Acetylbetulinsäure (ca. 0,002 mol) nach dem Verfahren gemäß 1)b) erhaltene Säurechlorid wird ohne weitere Reinigung umgesetzt. Dazu wird es in 35 ml wasserfreiem Dioxan gelöst und eine äquimolare Menge Tris-(hydroxymethyl)-aminomethan (0,002, 0,25g) wird zugegeben. Nach Zugabe einer Spatelspitze DMAP und von 3 Tropfen Pyridin wird das Reaktionsgemisch für 8 h auf 80°C erhitzt. Anschließend wird die Reaktionslösung am Rotationsverdampfer eingeengt und der Rückstand in Chloroform aufgenommen. Diese Chloroformlösung wird mehrmals mit 1%iger Salzsäure, Wasser und gesättigter Kochsalzlösung pyridinfrei gewaschen. Nach Trocknung über Na₂SO₄ wird das Lösungsmittel abgezogen und das Produkt über eine Kieselgelsäule oder (und) Chromatotron gereinigt. Als Laufmittel wurde ein Chloroform-Methanol-Gemisch im Verhältnis 10:1 eingesetzt. Ausbeute 15%, Fp.: 184°C

Im Folgenden wird der Einsatz der erfindungsgemäßen Verbindungen zur Wachstumsinhibierung von Tumoren im Vergleich mit Betulinsäure (Vergleichsverbindung A) beschrieben.

### Wachstumsinhibierung in verschiedenen Tumorlinien:

Beispiele 1 & 2 zeigen eine direkten Vergleich von Vergleichsverbindung A (Betulinsäure) mit einem neuartigen Derivat (Verbindung B) in gepaarten Wachstumsassays (Zellzahlabnahme nach 3 Tagen nach einmaliger Applikation der angegebenen Konzentrationen der entsprechenden Verbindungen am Tag 1).

Beispiel 1: Vergleich von Verbindung (A) (Betulinsäure) mit Verbindung (B) (geschützter Betulinsäure-Trisester) - Inhibierung des Wachstums in der Melanomlinie 518A2 (erhalten von Peter Schrier, Leiden, Niederlande) nach 48 Std. Die X-Achse gibt die Konzentration der Verbindungen A bzw. B in µg/ml an, die Y-Achse die Überlebensrate der Zellen in % der unbehandelten Kontrollen.
Die ED50 für Vergleichsverbindung (A) liegt in der Grössenordnung von 5 µg/ml, für die Verbindung (B) bei etwa 0.7 µg/ml.

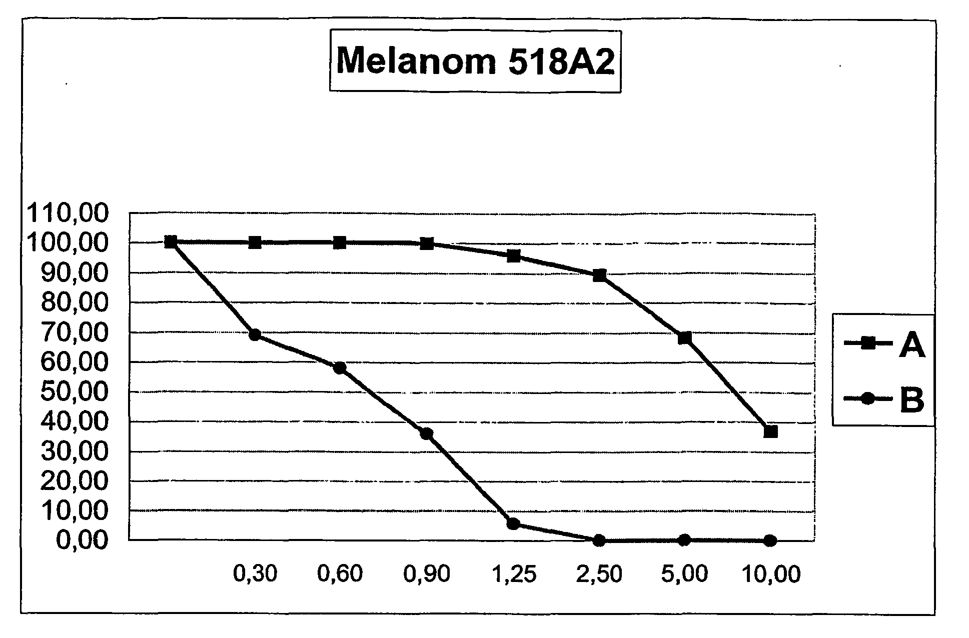

### Verbindung (B):

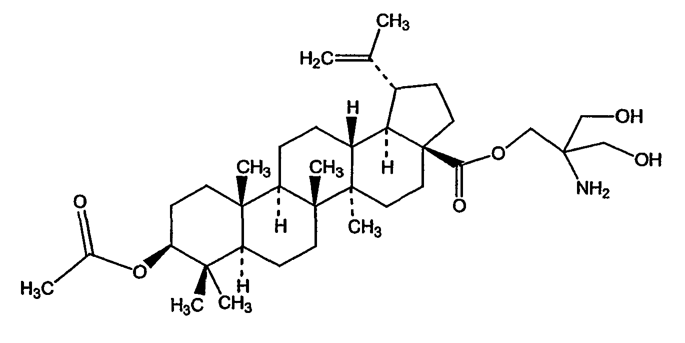

Die Verbindung (B) zeigt somit eine deutlich höhere Wirksamkeit als die Vergleichsbindung (A) in einer Grössenordnung von bis zu 7 - fach an.

Eine ähnlich bessere Wirksamkeit zeigt sich auch bei Sarkomen, wie nachfolgend exemplarisch an einer Zelllinie gezeigt wird.

**Beispiel 2:** Vergleich der Wirksamkeit von Vergleichsverbindung (A) (Betulinsäure) mit Verbindung (B) (geschützter Betulinsäure-Trisester) in einer Liposarkomlinie (ATCC HTB-92). Die X- Achse gibt die Konzentration der Verbindungen A bzw. B in µg/ml an, die Y-Achse die Überlebensrate der Zellen in % der unbehandelten Kontrollen.

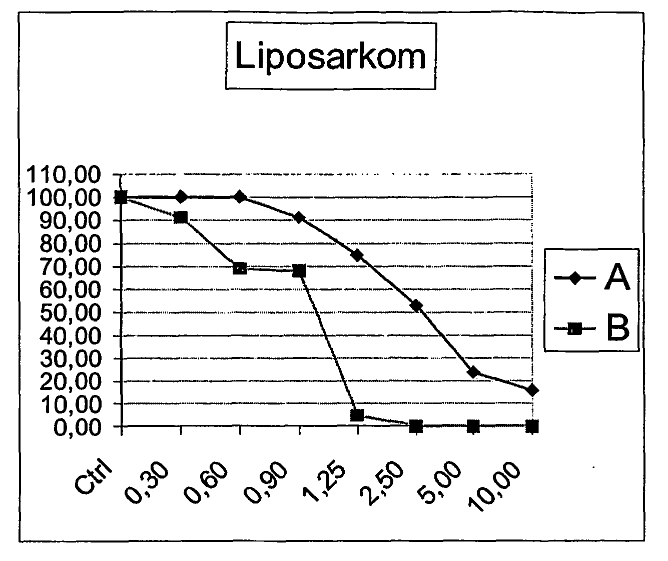

Die ED50 für die Vergleichsverbindung (A) beträgt 2,5 µg/ml und 0,9 µg/ml für die Verbindung (B).

**Beispiel 3:** Vergleich der Wirkung verschiedener erfindungsgemäßer Betulinsäurederivate bzw. der Vergleichsverbindung (Betulinsäure) auf verschiedene Melanom-Sarkomlinien. Es handelt sich jeweils um Angaben des Zellüberlebens in Prozent (Mittelwerte) verglichen mit den unbehandelten Zellkulturen.

### 3a) Vergleichsverbindung (A) (Betulinsäure) - Wirkung auf Liposarkomlinie ATCC HTB-92

| Konz. µg/ml. | Überleben in % |
|---|---|
| 0,3 | 98,0 |
| 0,6 | 92,8 |
| 1 | 92,3 |
| 1,25 | 79,5 |
| 2,5 | 49,3 |
| 5 | 20,8 |
| 10 | 15,7 |

### 3b) Verbindung (B) (geschützter Betulinsäure-Trisester) - Wirkung auf Liposarkomlinie ATCC HTB-92

| Konz. µg/ml. | Überleben in % |
|---|---|
| 0,3 | 89,5 |
| 0,6 | 70,7 |
| 1 | 12,0 |
| 1,25 | 2,9 |
| 2,5 | 3,0 |
| 5 | 0,0 |
| 10 | 0,0 |

### 3c) Verbindung (B) (geschützter Betulinsäure-Trisester) - Wirkung auf Melanomlinie 518A2

| Konz. µg/ml. | Überleben in % |
|---|---|
| 0,3 | 83,0 |
| 0,6 | 56,3 |
| 1 | 11,3 |
| 1,25 | 10,3 |
| 2,5 | 2,8 |
| 5 | 0,0 |
| 10 | 0,0 |

### 3d) Verbindung (C) (geschütztes Betulinsäure-Trisamid) - Wirkung auf Melanomlinie 518A2 & Liposarkomlinie ATCC HTB-92

### Verbindung (C):

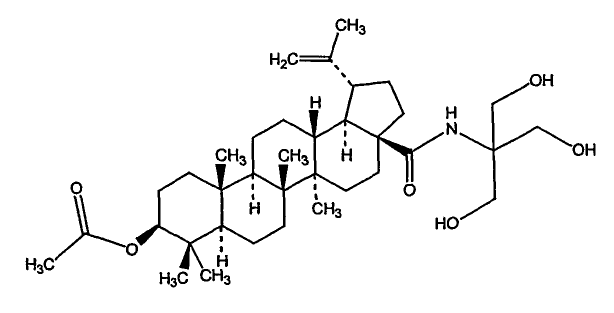

| Konz. µg/ml. | Überleben in % Melanom |
|---|---|
| 0,25 | 96 |
| 0,5 | 93 |
| 1 | 67,5 |

| Konz. µg/ml. | Überleben in % Liposarkom |
|---|---|
| 0,3 | 96 |
| 0,6 | 82 |
| 1,0 | 81 |

**Beispiel 4:** Herstellung einer Einschlussverbindung umfassend Verbindung (B) (geschützter Betulinsäure-Trisester) in HBC (2-Hydroxpropyl-beta-cyclodextrin, Fa. Sigma, Kat.Nr. H-107) oder in HGC (2-Hydroxypropyl-gamma-cyclodextrin, Fa. Sigma, Kat.Nr. H-125).

### Verbindung (B):

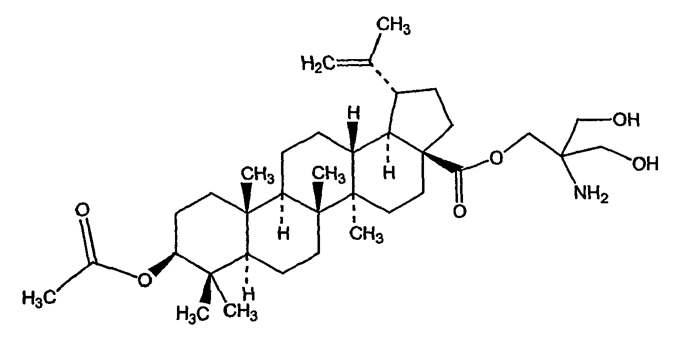

Im folgenden sind die jeweiligen Stocklösungen für die weitere Verdünnung angeführt. Verbindung (B) wurde jeweils zu 50 oder 100 mg/ml in EtOH gelöst. HBC wurde separat in Wasser oder Wasser plus EtOH gelöst. Die weitere Vorgangsweise war im Prinzip wie im mitgelieferten Datenblatt von Sigma beschrieben. Es wurde nicht versucht den pH-Wert einzustellen. Die Löslichkeit von Verbindung (B) in wässrigen Lösungen mittels Einschluss in HBC oder ähnlichen Verbindungen ist sicherlich noch weiter zu verbessern, z.B. in gamma-Cyclodextrine.

| | | | |
|---|---|---|---|
| Versuch | #1: | Verbindung (B) | 50 mg/ml in EtOH wie üblich gelöst |
| | #2: | Verbindung (B) | 5,9 mg/ml in 266 mg/ml HBC in H₂O |
| | #3: | Verbindung (B) | 20 mg/ml in 266 mg/ml HBC in 50% EtOH / |
| | | | 50% H₂O |
| | #4: | Verbindung (B) | 25 mg/ml in 200 mg/ml HGC in 50% EtOH / |
| | | | 50% H₂O |

Die oben angeführten Stocklösungen wurden auf 1 mg/ml in EtOH verdünnt und anschliessend direkt dem Kulturmedium zugegeben.

### 4a) Verbindung (B) (geschützter Betulinsäure-Trisester) als Einschlussverbindung in HBC (2-Hydroxpropyl-beta-cyclodextrin) oder in HGC (2-Hydroxypropyl-gamma-cyclodextrin)- Wirkung auf Melanomlinie 518A2:

Überleben in % wie bei allen anderen Daten nach 3 Tagen Zellkultur bei einmaliger Applikation am Tag 1

### Versuch #1

| Konz. µg/ml. | Überleben in % Melanom |
|---|---|
| 1,0 | 50 |
| 2,0 | 9,5 |
| 3,0 | 0,0 |
| 4,0 | 0,0 |

### Versuch #2

| Konz. µg/ml. | Überleben in % Melanom |
|---|---|
| 1,0 | 65 |
| 2,0 | 25 |
| 3,0 | 0,0 |
| 4,0 | 0,0 |

### Versuch #3

| Konz. µg/ml. | Überleben in % Melanom |
|---|---|
| 1,0 | 77 |
| 2,0 | 24 |
| 3,0 | 0,0 |
| 4,0 | 0,0 |

### Versuch #4

| Konz. µg/ml | Überleben in % Melanom |
|---|---|
| 1,0 | 59 |
| 2,0 | 4 |
| 3,0 | 0,0 |

### 4b) Verträglichkeit von Verbindung (B) (geschützter Betulinsäure-Trisester) als Einschlussverbindung in HGC (2-Hydroxypropyl-gamma-cyclodextrin) in einem Mausmodell:

Methodik: Pathogenfreie weibliche C.B.-17 *scid*/*scid* (SCID) Mäuse (4-6 Wochen alt, Harlan Winkelmann, Borchen, Deutschland) wurden intravenös in die Schwanzvene mit einem in Wasser gelösten HGC/Verbindung (B) Komplex injiziert. Die Einschlussverbindung wurde wie in Beispiel 4 beschrieben hergestellt. Der Alkoholanteil wurde durch Gefriertrocknung des Gemisches und anschließender Rekonstitution in Wasser entfernt. Das Gesamtvolumen pro Injektion für die unten angeführten Experimente betrug 200 µl.

### Versuch # 1

Es wurden zuerst jeweils zwei Mäuse 3 mal hintereinander an jedem 3. Tag mit 100 µg HGC/Verbindung (B) Komplex behandelt. Bei guter Verträglichkeit wurde das nächste Paar von Mäusen mit der nächst höheren Dosis behandelt, wie in dem unteren Schema angeführt.

### Schema:

| | |
|---|---|
| Gruppe A: | 3 mal 100 µg HGC/Verbindung (B) Komplex |
| Gruppe B | 3 mal 200 µg HGC/Verbindung (B) Komplex |
| Gruppe C | 3 mal 400 µg HGC/Verbindung (B) Komplex, |

anschließend weiter mit der zuerst behandelten Gruppe A:

| | |
|---|---|
| Gruppe A | 3 mal 800 µg HGC/Verbindung (B) Komplex |
| Gruppe B | 3 mal 1500 µg HGC/Verbindung (B) Komplex; |

dann Abbruch wegen lokaler Reizungserscheinungen

Der Abbruch der Versuche erfolgte bei 1500 µg HGC/Verbindung (B) Komplex wegen starker lokaler Reizungserscheinungen in einer von zwei Mäusen jedoch ohne offensichtliche systemische Toxizität. Insgesamt konnte eine Menge von 800 µg HGC/Verbindung (B) Komplex pro Injektion pro Maus ohne offensichtliche Toxizität verabreicht werden. Bei höheren Konzentrationen (etwa > 1,5 mg HGC/Verbindung (B) Komplex pro Maus) traten lokale Reizungserscheinen auf. Es ist denkbar, dass eine langsamere Infusion diese Nebenwirkungen zu vermeiden hilft.

### Versuch #2

Es wurde in einer Versuchsgruppe von 3 Mäusen pro Maus jeweils 800 µg HGC/Verbindung (B) Komplex (in 200 µl Volumen) jeden 3. Tag gegeben, und das 6 mal hintereinander. Auch dies zeigte keine offensichtlichen klinisch-toxischen Effekte. Die anschließende Obduktion ergab keinen Hinweis auf makroskopische Organveränderungen.

## Patentansprüche

1. Neues Betulinsäurederivat mit der allgemeinen Formel (I) 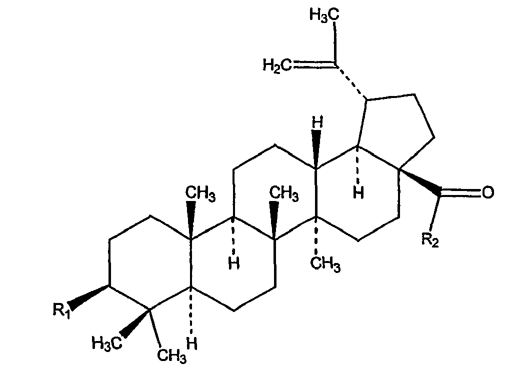 worin R₁ eine Hydroxygruppe, eine Aminogruppe, eine geschützte Hydroxygruppe oder eine geschützte Aminogruppe darstellt,
und R₂= 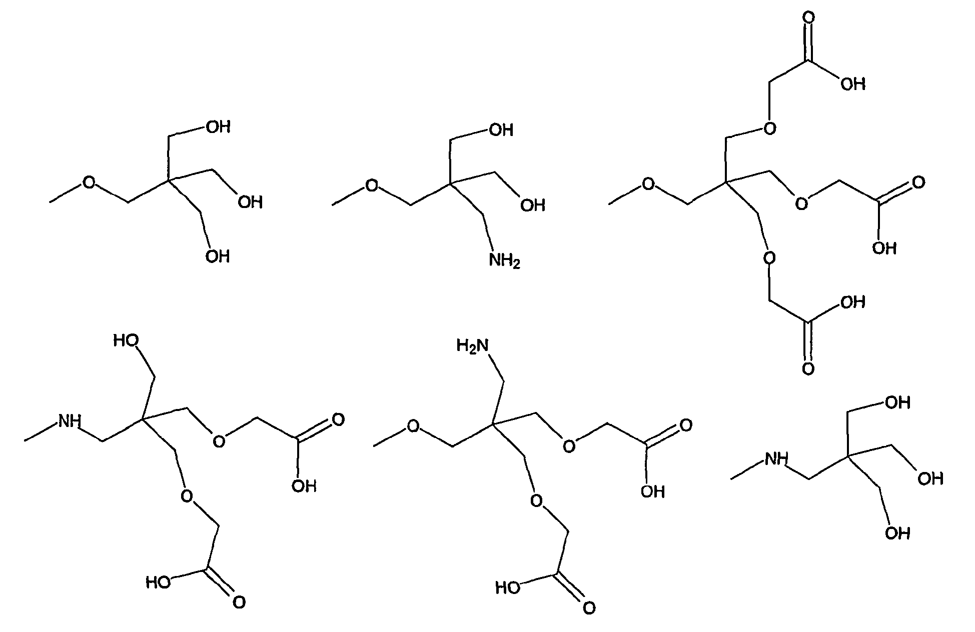 sowie deren Salze und Einschlussverbindungen.

2. Verbindung nach Anspruch 1, worin R₁ eine Hydroxygruppe, eine Aminogruppe oder eine der folgenden geschützten Hydroxy- oder Aminogruppen ist: 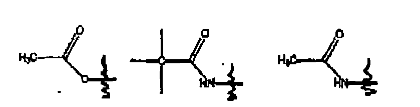 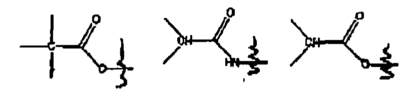 und R₂ die oben angegebene Bedeutung hat.

3. Verfahren zur Herstellung einer Verbindung nach Anspruch 1 oder 2, **dadurch gekennzeichnet, dass** ein durch den Substituenten R₁ entsprechend geschütztes Betulinsäurehalogenid mit einem zur Vorsehung des Substituenten R₂ entsprechend substituierten Alkohol oder Amin umgesetzt wird und die derart erhaltene Verbindung der allgemeinen Formel (I), worin R₁ eine geschützte Hydroxy- oder Aminogruppe darstellt, gewünschtenfalls entschützt wird, um eine Verbindung der allgemeinen Formel (I) vorzusehen, worin R₁ Hydroxy oder Amino bedeutet.

4. Verwendung einer Verbindung nach Anspruch 1 oder 2 zur Herstellung eines Medikaments.

5. Verwendung einer Verbindung nach Anspruch 1 oder 2 zur Herstellung eines Medikaments zur Wachstumsinhibierung von Tumoren wie Melanomen und neuroektodermalen Tumoren und/oder zur Behandlung von Sarkomen und HIV in Säugem.

6. Verwendung einer Verbindung nach Anspruch 1 oder 2 zur Herstellung eines Medikaments zur Kombinationstherapie mit anderen zytostatisch wirksamen Substanzen und Substanzen die den Zelltod modulieren können, z.B. Antisensoligonukleotide gegen verschiedene anti-apoptotische Bcl-2 Familienmitglieder, insbesondere Bcl-2, Bcl-xL sowie Mcl-1.

7. Pharmazeutische Zusammensetzung umfassend eine Verbindung nach einem der Ansprüche 1 oder 2.

## Claims

1. A novel betulinic acid derivative having the general formula (I) 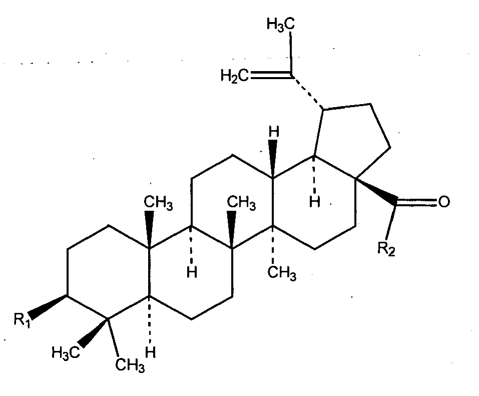 wherein R₁ represents a hydroxy group, an amino group, a protected hydroxy group or a protected amino group, and R₂ = 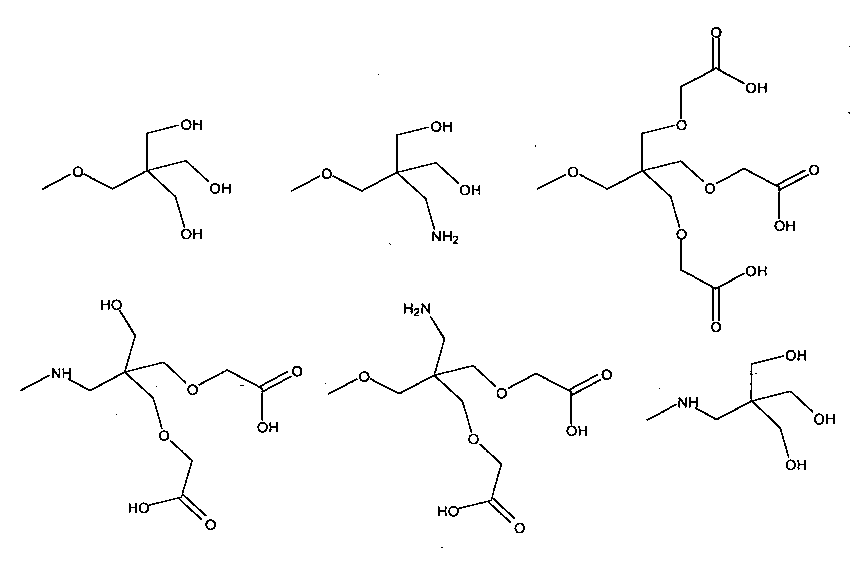 as well as its salts and inclusion compounds.

2. A compound according to claim 1, wherein R₁ represents a hydroxy group, an amino group or one of the following protected hydroxy or amino groups: 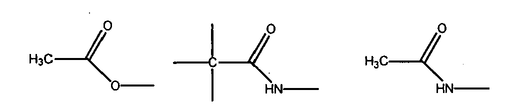 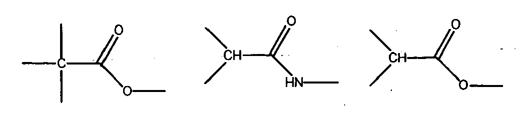 and R₂ is as defined above.

3. A method for preparing a compound according to claim 1 or 2, **characterized in that** a betulic acid halide appropriately protected by the substituent R₁ is reacted with an alcohol or amine appropriately substituted to provide the substituent R₂ and the thus obtained compound of the general formula (I), wherein R₁ represents a protected hydroxy or amino group, is deprotected, if desired, to provide a compound of the general formula (I) wherein R₁ represents hydroxy or amino.

4. The use of a compound according to claim 1 or 2 for the preparation of a medicament.

5. The use of a compound according to claim 1 or 2 for the preparation of a medicament for inhibiting the growth of tumors like melanomas and neuroectodermal tumors, and/or for treating sarcomas and HIV diseases in mammals.

6. The use of a compound according to claim 1 or 2 for the preparation of a medicament for a combination therapy with other cytostatically active substances and cell-death-modulating substances, e.g., antisensoligonucleotides against various anti-apoptotic Bcl-2 family members and, in particular, Bcl-2, Bcl-xL as well as Mcl-1.

7. A pharmaceutical composition comprising a compound according to any one of claims 1 or 2.

## Revendications

1. Nouveau dérivé de l'acide bétulinique de formule générale (I) 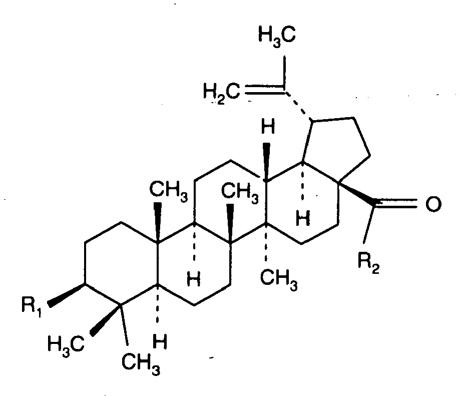 où R₁ représente un groupe hydroxy, un groupe amino, un groupe hydroxy protégé ou un groupe amino protégé,
et R₂ = 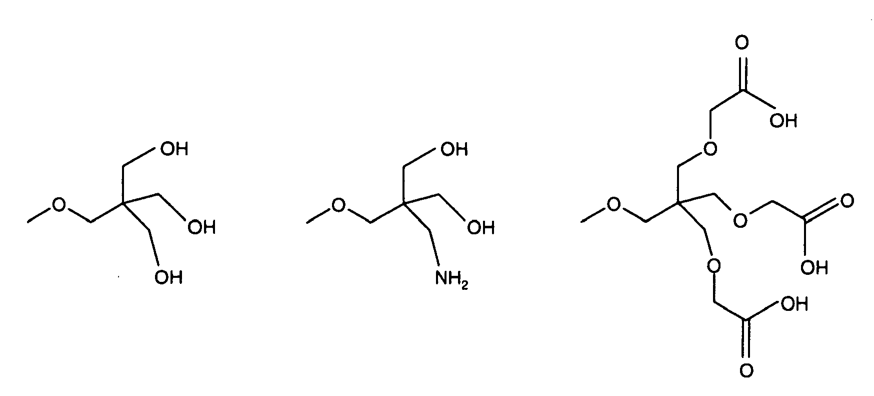 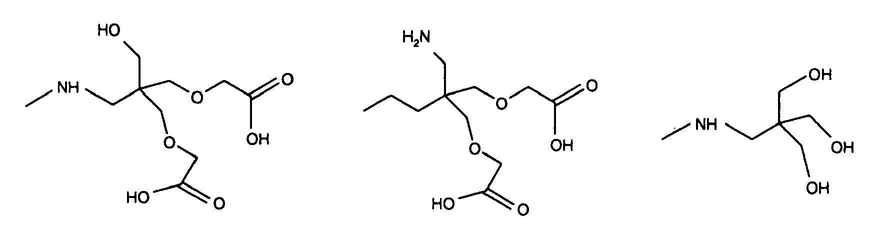 ainsi que ses sels et ses composés d'inclusion.

2. Composé selon la revendication 1, où R₁ est un groupe hydroxy, un groupe amino ou un des groupes hydroxy ou amino protégés suivants : 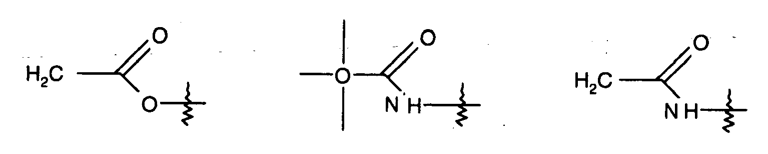 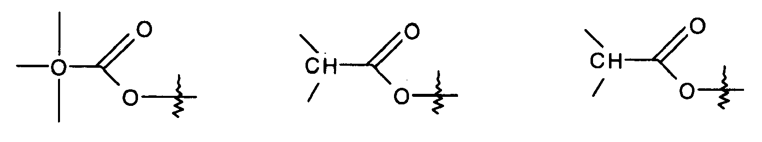 et R₂ a la signification indiquée ci-dessus.

3. Procédé de préparation d'un composé selon la revendication 1 ou 2, **caractérisé en ce qu'**un halogénure d'acide bétulinique protégé de façon correspondante par le substituant R₁ est mis à réagir avec un alcool ou une amine substitués de façon correspondante en vue de la prévision du substituant R₂ et le composé ainsi obtenu de formule générale (I) où R₁ représente un groupe hydroxy ou un groupe amine protégés, est déprotégé le cas échéant pour prévoir un composé de formule générale (I) où R₁ représente un groupe hydroxy ou amino.

4. Utilisation d'un composé selon la revendication 1 ou 2 pour la préparation d'un médicament.

5. Utilisation d'un composé selon la revendication 1 ou 2 pour la préparation d'un médicament destiné à l'inhibition de la croissance de tumeurs telles que mélanomes et tumeurs neuroectodermiques et/ou au traitement de sarcomes et du VIH chez le mammifère.

6. Utilisation d'un composé selon la revendication 1 ou 2 pour la préparation d'un médicament en vue d'un traitement associatif avec d'autres substances à effet cytostatique et des substances susceptibles de moduler la mort cellulaire, par exemple des oligonucléotides antisens dirigés contre divers membres de la famille des anti-apoptotiques Bcl-2, notamment Bcl-2, Bcl-xL ainsi que Mcl-1.

7. Composition pharmaceutique comprenant un composé selon une des revendications 1 ou 2.
